# EUROPEAN PATENT APPLICATION

(11) **EP 3 904 884 A1**
(43) Date of publication of application: **03.11.2021**
(21) Application number: 20172395.4
(22) Date of filing: 30.04.2020
(51) Int. Cl.: G01N 33/68

(54) **METHOD FOR PREDICTING THE COURSE OF A VIRAL DISEASE**

(71) Applicant: Heinrich-Pette-Institut, 20251 Hamburg (DE)
(72) Inventor: GABRIEL, Gülsah, 20251 Hamburg (DE); STANELLE-BERTRAM, Stephanie, 20251 Hamburg (DE)
(74) Representative: Uexküll & Stolberg

(57) **Abstract**

The invention relates to a method for predicting the course of a viral disease in a male subject infected with an influenza virus or coronavirus which is based on measuring testosterone and/or estradiol levels in said subject. The invention further relates to a method for monitoring the course of a viral disease in a male subject infected with an influenza virus or coronavirus which comprises predicting the course of the disease in said subject and assigning the subject to preventive or therapeutic measures if a severe course of said viral disease is to be expected. The invention further relates to an aromatase inhibitor for use in a method of treating or preventing a severe course of a viral disease in a male subject infected with an influenza virus or coronavirus, wherein said subject has decreased testosterone levels and/or increased estradiol levels as compared to reference values. Finally, the invention also relates to a kit for carrying out one of the aforementioned methods.

## Description

The invention relates to a method for predicting the course of a viral disease in a male subject infected with an influenza virus or coronavirus which is based on measuring testosterone and/or estradiol levels in said subject. The invention further relates to a method for monitoring the course of a viral disease in a male subject infected with an influenza virus or coronavirus which comprises predicting the course of the disease in said subject and assigning the subject to preventive or therapeutic measures if a severe course of said viral disease is to be expected. The invention further relates to an aromatase inhibitor for use in a method of treating or preventing a severe course of a viral disease in a male subject infected with an influenza virus or coronavirus, wherein said subject has decreased testosterone levels and/or increased estradiol levels as compared to reference values. Finally, the invention also relates to a kit for carrying out one of the aforementioned methods.

### FIELD OF THE INVENTION

Influenza can sometimes lead to severe disease progression with high mortality. In cases with a severe course of disease, patients may have to be treated in intensive care units (ICUs). Approximately 30% of all patients undergoing intensive care for influenza develop severe respiratory complications, in particular the acute respiratory distress syndrome (ARDS) which lead to lung failure. Severe respiratory complications can occur very rapidly in influenza patients, sometimes within only a few hours.

Similarly, ARDS is also regularly observed in a subgroup of patients which are infected with a coronavirus, in particular with the severe acute respiratory syndrome coronavirus (SARS-CoV) or severe acute respiratory syndrome coronavirus 2 (SARS-CoV-2). While about 80% of the people infected with SARS-CoV-2 recover without special treatment, about 6% of the infected people encounter severe respiratory complications, including ARDS. Elderly people and those with pre-existing conditions such as asthma, diabetes or heart disease have an increased risk of a severe course. Again, the development of severe respiratory complications can occur very fast.

So far, it is not possible to reliably predict whether a patient who is infected with an influenza virus or coronavirus will develop severe respiratory complications like ARDS. Accordingly, there is a need for new prognostic methods that enable physicians to identify patients with a particular high risk of developing a severe course of disease. Such prognostic methods would allow assigning such patients to specific treatments even before the onset of respiratory complications, thereby significantly improving their chance of survival.

### DESCRIPTION OF THE INVENTION

The studies underlying the present invention have revealed that the determination of the testosterone and/or estradiol levels in a body fluid sample of a subject, preferably in a serum sample, allows predicting whether an infectious disease which is caused by infection with an influenza virus or coronavirus takes a severe or moderate course.

Specifically, it has been found by retrospective analysis that the testosterone levels which can be detected in samples from male patients infected with an influenza virus or coronavirus are significantly lower in patients that show a severe course of disease at a later stage, including severe respiratory complications like ARDS. At the same time, the estradiol levels are higher in patients that later on show complications.

Based on this insight, the present invention allows providing tests that reliably predict, based on testosterone and/or estradiol levels, whether an influenza virus or coronavirus infection takes a severe course that is likely to require intensive care measurements like artificial respiration. In this way, the methods of the invention allow an improved risk analysis in hospitals and intensive care units.

Thus, in a first aspect the present invention provides a method for predicting the course of a viral disease in a male subject infected with an influenza virus or coronavirus, said method comprising:
(a) providing a body fluid sample from the subject that is infected with said influenza virus or coronavirus;
(b) determining in said sample the concentration of testosterone and/or estradiol; and
(c) comparing the concentration obtained in step (b) with at least one testosterone and/or estradiol reference value;
wherein the comparison of the concentration obtained in step (b) with said at least one reference value indicates whether a severe course of said viral disease is to be expected in said subject.

In step (a) of the above method, a body fluid sample obtained from the infected male subject is provided. The sample to be used in the above method can be, in principle, any type of body fluid obtained from the subject to be diagnosed. In a preferred aspect, the sample will be a blood sample, such as a whole-blood sample, or a plasma or serum sample. In an even more preferred aspect, the sample will be a serum sample, such as a human serum sample.

The sample originates from a male subject that has already been diagnosed to be infected with an influenza virus or coronavirus. The male subject can be an adult between 18 and 120 years old, but it will be preferred that the subject is at least 20 years old, at least 25 years old, at least 30 years old, at least 35 years old, at least 40 years old, at least 45 years old, at least 50 years old, at least 55 years old, or at least 60 years old.

The influenza virus or coronavirus diagnosis can be obtained from any method suitable for confirming the presence of a virus in the subject, for example by PCR-based detection of virus-specific nucleic acid, by electron microscopy, by detection of antibodies against viral proteins, or by immunodetection of viral components using conjugated antibodies, e.g. in the form of an enzyme-linked immunosorbent assay (ELISA). In a preferred aspect, the influenza virus or coronavirus diagnosis in the subject has been obtained by an ELISA.

As used herein, the term influenza virus relates to a group of RNA viruses that cause the infectious disease influenza. Common symptoms of influenza include fever, headaches, and fatigue. These symptoms are caused by large amounts of pro-inflammatory cytokines and chemokines that are released by influenza-infected cells, including interferon or tumor necrosis factor (TNF). It has been proposed that the massive release of cytokines can produce a life-threatening cytokine storm. The methods described herein can be used to predict such a severe course of disease. These methods can be applied to patients infected with any influenza subtype, including influenza A subtypes H1N1, H2N2, H3N2, H5N1, H7N7, H1N2, H9N2, H7N2, H7N3, H10N7, H7N9, and influenza B subtypes of the lines Victoria, Yamagata, Yamaguchi, Yokohama, Yunnan, Zhuhai.

The term coronavirus relates to a group of related viruses that cause diseases in mammals and birds. In humans, coronaviruses cause respiratory tract infections that can be linked with symptoms ranging from mild to severe. Mild infections are cause symptoms similar to those of a common cold. More severe coronavirus infections can cause life-threatening complications like the Severe Acute Respiratory Syndrome (SARS), the Middle East Respiratory Syndrome (MERS) and the Coronavirus disease 2019 (COVID-19). According to the invention, the subject can be infected with any type of a coronavirus, including viruses of the genus Alphacoronavirus, Betacoronavirus, Gammacoronavirus and Deltacoronavirus, but it will preferably be a coronavirus that is known to cause respiratory infections, such as SARS, MERS and COVID-19. It is particularly preferred that the subject is infected with the severe acute respiratory syndrome coronavirus (SARS-CoV) or the severe acute respiratory syndrome coronavirus 2 (SARS-CoV-2).

In step (b) of the above method, the concentration of testosterone and/or estradiol is determined in the sample from the infected male patient. In one embodiment, the concentration of testosterone is determined in the sample from the infected patient. Testosterone is the primary male sex hormone and plays a key role in the development of male reproductive tissues such as testes and prostate. Testosterone is a steroid from the androstane class which is synthesized in several steps from cholesterol. In males, testosterone is secreted primarily by the testicles. In females, which normally have testosterone levels that are 7-8 times lower compared to males, testosterone is produced in the ovaries.

Methods for determining the concentration of testosterone are well known in the art and have been described in the scientific literature, for example, in van Nuland et al. (2019), Star-Weinstock & Dey (2019), Wooding et al (2015), and Ankarberg-Lindgren at al. (2018). In addition, kits are commercially available for testosterone quantification in a sample, such as the Testosterone ELISA Assay Kit (Eagle Biosciences, Amherst, USA) or the Testosterone ELISA Kit (Abcam, Berlin, Germany).

In another embodiment, the concentration of estradiol is determined in the sample from the infected male patient. Estradiol, which is also referred to as E2 in the literature, is an estrogen steroid hormone and the major female sex hormone. As such, it is involved in the regulation of the estrous and menstrual female reproductive cycles. Estradiol is mandatory for development and maintenance of female reproductive tissues such as the mammary glands, uterus, and vagina during puberty, adulthood, and pregnancy. Estradiol is produced from cholesterol through a series of reactions and intermediates. In females, the production takes place especially in the follicles of the ovaries. In males, estradiol is mainly produced by catalytic conversion of testosterone, a reaction that is catalyzed by the enzyme aromatase.

Methods for determining the concentration of estradiol are well known in the art and have been described in the scientific literature, for example, in Wooding et al. (2015), Siqueira Ferreira et al. (2017), and Keski-Rahkonen et al. (2015), Analytical Chemistry, 87, 14, 7180-7186. In addition, kits are commercially available for testosterone quantification in a sample, such as the Estradiol Parameter Assay Kit, (R&D Systems, Inc., Minneapolis, USA), the Estradiol ELISA Kit (Eagle Biosciences, Amherst, USA) or the Human Estradiol E2 ELISA Kit (Abcam, Berlin, Germany). It is particularly preferred that step (b) of the above method comprises the determination of both the testosterone concentration and the estradiol concentration in the sample from the infected male patient. The testosterone concentration and the estradiol concentration can be determined in the same or in different aliquots of the sample, in either order.

Once the concentration of testosterone and/or estradiol has been determined in step (b) of the above method, the concentration is compared with at least one testosterone and/or estradiol reference value. The comparison of the testosterone and/or estradiol concentration measured in the sample with at least one reference value indicates whether a severe course of said viral disease is to be expected in said subject.

In one preferred embodiment, the method of the first aspect of the invention comprises in step (b) the determination of the testosterone concentration in the body fluid sample, in particular a blood or serum sample, and step (c) comprises the comparison of the testosterone concentration of the sample with a testosterone reference value, wherein a severe course of disease is to be expected if the concentration obtained in step (b) falls below the reference value.

In males between 18-50 years, a concentration of between 8.69 to 29.00 nMol testosterone per liter blood serum is considered normal. Instead, testosterone concentrations values below 8.69 nMol/l are considered less than normal in males of that age and therefore indicative for potentially severe complication in patients infected with influenza virus or coronavirus. Therefore, in one embodiment, the reference value for adult males of that age is 8.69 nMol/l and a severe course of disease is to be expected if the concentration in the sample falls below 8.69 nMol/l. In another embodiment, the reference value for males at that age is 8.5 nMol/l and a severe course of disease is to be expected if the concentration in the sample falls below 8.5 nMol/l. In yet another embodiment, reference value for males at that age is 7.5 nMol/l and a severe course of disease is to be expected if the concentration in the sample falls below 7.5 nMol/l. In yet another embodiment, reference value for males at that age is 6.5 nMol/l and a severe course of disease is to be expected if the concentration in the sample falls below 6.5 nMol/l. In yet another embodiment, reference value for males at that age is 5.5 nMol/l and a severe course of disease is to be expected if the concentration in the sample falls below 5.5 nMol/l. In yet another embodiment, reference value for males at that age is 4.5 nMol/l and a severe course of disease is to be expected if the concentration in the sample falls below 4.5 nMol/l. In yet another embodiment, reference value for males at that age is 3.5 nMol/l and a severe course of disease is to be expected if the concentration in the sample falls below 3.5 nMol/l. In yet another embodiment, reference value for males at that age is 2.5 nMol/l and a severe course of disease is to be expected if the concentration in the sample falls below 2.5 nMol/l. In yet another embodiment, reference value for males at that age is 1.5 nMol/l and a severe course of disease is to be expected if the concentration in the sample falls below 1.5 nMol/l.

In males older than 51 years, a concentration of between 6.68 to 25.8 nMol testosterone per liter blood serum is considered normal. Instead, testosterone concentrations values below 6.68 nMol/l are considered less than normal in males of that age and therefore indicative for potentially severe complication in patients infected with influenza virus or coronavirus. Therefore, in one embodiment, the reference value for adult males of that age is 6.68 nMol/l and a severe course of disease is to be expected if the concentration in the sample falls below 6.68 nMol/l. In yet another embodiment, reference value for males at that age is 6.5 nMol/l and a severe course of disease is to be expected if the concentration in the sample falls below 6.5 nMol/l. In yet another embodiment, reference value for males at that age is 5.5 nMol/l and a severe course of disease is to be expected if the concentration in the sample falls below 5.5 nMol/l. In yet another embodiment, reference value for males at that age is 4.5 nMol/l and a severe course of disease is to be expected if the concentration in the sample falls below 4.5 nMol/l. In yet another embodiment, reference value for males at that age is 3.5 nMol/l and a severe course of disease is to be expected if the concentration in the sample falls below 3.5 nMol/l. In yet another embodiment, reference value for males at that age is 2.5 nMol/l and a severe course of disease is to be expected if the concentration in the sample falls below 2.5 nMol/l. In yet another embodiment, reference value for males at that age is 1.5 nMol/l and a severe course of disease is to be expected if the concentration in the sample falls below 1.5 nMol/l. In yet another embodiment, reference value for males at that age is 1.0 nMol/l and a severe course of disease is to be expected if the concentration in the sample falls below 1.0 nMol/l.

For estradiol, a concentration of between 27.1 and 52.2 pg estradiol per milliliter blood serum is considered normal in males, independent from their age. Instead, estradiol concentrations values above 52.2 pg/ml are considered more than normal and therefore indicative for potentially severe complication in males infected with influenza virus or coronavirus. Therefore, in one embodiment, the reference value for adult males is 52.2 pg/ml and a severe course of disease is to be expected if the estradiol concentration in the sample exceeds 52.2 pg/ml. In another embodiment, the reference value for adult males is 55 pg/ml and a severe course of disease is to be expected if the estradiol concentration in the sample exceeds 55 pg/ml. In another embodiment, the reference value for adult males is 60 pg/ml and a severe course of disease is to be expected if the estradiol concentration in the sample exceeds 60 pg/ml. In another embodiment, the reference value for adult males is 70 pg/ml and a severe course of disease is to be expected if the estradiol concentration in the sample exceeds 70 pg/ml. In yet another embodiment, the reference value for adult males is 80 pg/ml and a severe course of disease is to be expected if the estradiol concentration in the sample exceeds 80 pg/ml. In yet another embodiment, the reference value for adult males is 90 pg/ml and a severe course of disease is to be expected if the estradiol concentration in the sample exceeds 90 pg/ml. In yet another embodiment, the reference value for adult males is 100 pg/ml and a severe course of disease is to be expected if the estradiol concentration in the sample exceeds 100 pg/ml.

The above method can be used as a tool for patient surveillance. Accordingly, in a second aspect, the invention provides a method for monitoring the course of a viral disease in a male subject infected with an influenza virus or coronavirus, said method comprising:
(a) repeatedly conducting a method according to the first aspect of the invention defined above in predefined time intervals, and
(b) assigning the subject to preventive or therapeutic measures if based on the results obtained in step (a) a severe course of the viral disease is to be expected.

The predictive method according to the first aspect of the invention can be used for monitoring the course of a viral disease in a male subject infected with an influenza virus or coronavirus. Since subjects infected with influenza or coronavirus may encounter complications rather rapidly, is it helpful to predict the further course of disease in predefined time intervals, such as every 24 hours, every 18 hours, every 12 hours, or every 6 hours. A severe course of the infection with respiratory complications can be expected if a drop in the testosterone levels and/or an increase in the estradiol levels can be observed. In this case, a severe course of disease is likely and the subject can be assigned to preventive or therapeutic measures. Such measures include an increased clinical surveillance, the initiation of artificial respiration, or the administration of anti-viral drugs like remdesivir. The measures may also include the treatment of the patient with one or more aromatase inhibitors as described elsewhere herein, the treatment of the patient with one or more testosterone or testosterone derivative as described elsewhere herein, or the combinations of such therapies. In the method of the second aspect of the invention, a severe course of the viral disease may include the development of ARDS.

The observation that patients with decreased testosterone levels and/or increased estradiol levels regularly exert a higher risk to experience a severe course of diseases after infection with influenza virus or coronavirus suggests that these patients have increased amounts and/or increased activity levels of the aromatase enzyme which catalyzes the conversion of testosterone into estradiol. In these patients, the inhibition of aromatase may have a therapeutic effect.

Thus, in a third aspect, the invention provides an aromatase inhibitor for use in a method of treating or preventing the severe course of a viral disease in a male subject infected with an influenza virus or coronavirus, wherein said subject has (a) decreased testosterone levels compared to the normal reference levels discussed above and/or (b) increased estradiol levels compared to normal reference levels discussed above. The aromatase inhibitor will be formulated to be compatible with the intended route of administration. Different routes of administration are feasible for providing the aromatase inhibitor to the subject. Preferably, the aromatase inhibitor is formulated for oral administration, e.g. in the form of tablets, capsules, granule, powder, liquids, and the like. Alternatively, the aromatase inhibitor can be formulated for parenteral administration, for example, for intravenous or subcutaneous administration. The aromatase inhibitor may also be formulated for being administered by implantation, e.g. by admixing the aromatase inhibitor with a three-dimensional carrier or scaffold, such as a hydrogel.

Suitable aromatase inhibitors for use herein include, but are not limited to, aminoglutethimide, testolactone, anastrozole, letrozole, exemestane, vorozole, formestane, and fadrozole. Preferably, the aromatase inhibitor is for use in a method of treating or preventing a severe course of a viral disease which includes the development of ARDS.

Preferably, the administration of an aromatase inhibitor can be combined with testosterone supplementation. Accordingly, it is particularly preferred that testosterone is administered to the subject who receives the aromatase inhibitor. Testosterone administration and administration of the aromatase inhibitor can occur simultaneously or sequentially, in either order.

In a fourth aspect, the invention provides testosterone or a testosterone derivative for use in a method of treating or preventing the severe course of a viral disease in a male subject infected with an influenza virus or coronavirus, wherein said male subject has (a) decreased testosterone levels compared to the normal reference levels discussed above and/or (b) increased estradiol levels compared to normal reference levels discussed above. The reference values will be those discussed above in connection with the method according to the first aspect of the invention.

Again, the testosterone or testosterone derivative will be formulated to be compatible with the intended route of administration. Different routes of administration are feasible for providing testosterone or its derivative to the subject. Preferably, the testosterone or testosterone derivative is formulated for oral administration, e.g. in the form of tablets, capsules, granule, powder, liquids, and the like. Alternatively, the testosterone or testosterone derivative can be formulated for parenteral administration, for example, for intravenous or subcutaneous administration. However, it is preferred that the testosterone or testosterone derivative is formulated for transdermal or transmucosal application, e.g. in the form of a patch that releases testosterone to the skin.

Preferably, as stated above, the administration of testosterone can be combined with the administration of one or more aromatase inhibitors. It is hence preferred that the subject who receives the testosterone or testosterone derivative also receives one of the aromatase inhibitors referred to above. Testosterone administration and administration of the aromatase inhibitor can occur simultaneously or sequentially, in either order.

Finally, in a fifth aspect, the invention provides a kit for carrying out the methods described herein above, comprising:
(a) means for determining whether a subject is infected with an influenza virus or coronavirus;
(b) means for determining the concentration of testosterone and/or estradiol; and
(c) optionally, buffers and diluents.

In one embodiment, the kit contains antibodies which are useful for the detection of influenza virus or coronavirus antigens, e.g. by an ELISA. The kit may also include suitable immunologic reagents for determining the concentration of testosterone and/or estradiol.

### DESCRIPTION OF THE FIGURES

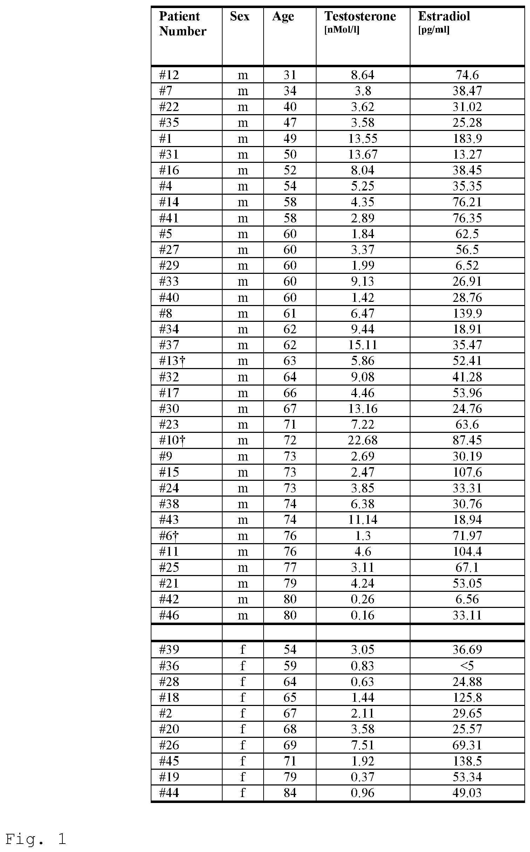

Figure 1 shows the testosterone and estradiol levels determined in a number of COVID-19 patients.
Figure 2 shows the results from total testosterone expression level measurements in H7N9 male infected with H7N9 influenza A virus.

### EXAMPLES

The invention is described in the following on the basis of examples, for the purpose of illustration, without limiting the invention. It will be evident to a person skilled in the art that modifications and variations of the examples described are possible without deviating from the idea of the invention.

### Example 1: Determination of hormone status in COVID-19 patients

45 COVID-19 patients at the University Hospital Hamburg Eppendorf requiring intensive care were examined. Among these patients, 35 were males and 10 were females. The median age within males and females was comparable with 62 and 67.5, respectively. Majority of the patients presented an elevated body mass index (BMI) (31.4% of males and 30% of females with a BMI ≥30). All patients presented comorbidities in males and females, such as adipositas (males 69%; females 50%), followed by diabetes type II (males 22.9%; females 20%), hypertension (males 45.7%, females 33.3%) and cancer (males 22.9%, females 33.3%). Acute respiratory distress (ARDS) detected was classified as moderate or severe in most male (37% or 26%) and female (33% or 33%) patients. Sequential organ failure assessment (SOFA) scores were evaluated in males and females presenting high (4-7) or very high (8-11) scores in males (35% or 25%) and females (40% or 60%). Due to the strong sex bias of males-to-females with a ratio of 3.5:1, sex hormones known to play a key role not only in fertility but also in innate and adaptive immunity were measured.

Results: The results are shown in Table 1. Total testosterone levels were reduced in 69% of males. Herein, 26% of males showed very low and 43% of males extremely low testosterone levels. In 60% of females, testosterone levels were increased to high (50%) or very high (10%) levels. Estradiol levels were elevated in male COVID-19 patients (46%), either to high (30%) or very high (16%) levels. Comparably, 60% of females also showed elevated estradiol concentration to high (40%) or very high (20%) levels. Thus, the vast majority of male COVID-19 patients have very low testosterone levels and very high estradiol levels. In contrast, female COVID-19 patients tend to have high testosterone and estradiol levels. A shift in sex hormones, as seen here in male patients, hints towards increased aromatase activity, i.e. the enzyme that converts testosterone to estradiol.

### Example 2: Determination of hormone status in H7N9 influenza patients

A total of n=44 avian H7N9 influenza positive cases of reproductive age (18-49 years) were enrolled with a median age of 42 years. A total of n=54 avian H7N9 influenza positive cases were included in those ≥50 year olds with the median age of 61 years. The male H7N9 cases accounted for 75% in the younger and 70% in the older age groups, which is consistent with previous epidemiological studies based on larger laboratory-confirmed H7N9 cohorts. Blood samples of H7N9 patients were collected within acute phases after illness onset.

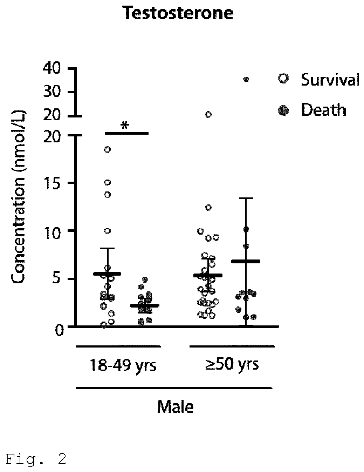

In order to assess the role of testosterone for the outcome of H7N9 infections, the testosterone concentrations were measured in all cohorts. Testosterone levels were strongly reduced in H7N9 infected men of both age groups assessed compared to virus negative H7N9 controls. Low testosterone levels strongly correlated with lethal outcome in H7N9 infected men in the age group of 18-49 year olds (P<0•001) (Figure 2). These data show that low testosterone levels in H7N9 infected men of 18-49 years of age correlate with an enhanced risk for lethal outcome.

### LITERATURE

1. Ankarberg-Lindgren et al. (2018), J Steroid Biochem Mol Biol, 183: 116-124.
2. Siqueira Ferreira et al. (2017), Journal of Chromatography B 1064, 109-114.
3. Star-Weinstock & Dey (2019), Clinical Mass Spectrometry, 13, 27-35.
4. Wooding et al (2015), Steroids, 96:89-94,
5. Van Nuland et al. (2019), J Pharm Biomed Anal., 170: 161-168.

## Claims

1. Method for predicting the course of a viral disease in a male subject infected with an influenza virus or coronavirus, said method comprising:
(a) providing a body fluid sample from the subject that is infected with said influenza virus or coronavirus;
(b) determining in said sample the concentration of testosterone and/or estradiol; and
(c) comparing the concentration obtained in step (b) with at least one testosterone and/or estradiol reference value;
wherein the comparison of the concentration obtained in step (b) with said at least one reference value indicates whether a severe course of said viral disease is to be expected in said subject.

2. Method according to claim 1, wherein said body fluid sample is a blood, plasma or serum sample.

3. Method according to any of claims 1-2, wherein said method comprises the comparison of the concentration obtained in step (b) with a testosterone reference value, wherein a severe course of said viral disease is to be expected if the concentration obtained in step (b) falls below the reference value.

4. Method according to any of claims 1-3, wherein said testosterone reference value is 8.69 nMol/l blood serum in males between 18-50 years or 6.68 nMol/l blood serum in males older than 51 years.

5. Method according to any of claims 1-2, wherein said method comprises the comparison of the concentration obtained in step (b) with an estradiol reference value, wherein a severe course of said viral disease is to be expected if the concentration obtained in step (b) exceeds the reference value.

6. Method according to any of claims 1-5, wherein said estradiol reference value is 52.2 pg/ml blood serum, more preferably 60 pg/ml blood serum.

7. Method according to any of claims 1-6, wherein said method comprises:
(i) the comparison of the testosterone concentration obtained in step (b) with a testosterone reference value, wherein said reference value preferably is 8.69 nMol/l blood serum in males between 18-50 years or 6.68 nMol/l blood serum in males older than 51 years, and
(ii) the comparison of the estradiol concentration obtained in step (b) with an estradiol reference value, wherein said reference value preferably is 52.2 pg/ml blood serum,
wherein a severe course of said viral disease is to be expected if the testosterone concentration obtained in step (b) falls below the testosterone reference value and the estradiol concentration obtained in step (b) exceeds the estradiol reference value.

8. Method according to any of claims 1-7, wherein said influenza virus is H7N9 or said coronavirus is SARS-CoV-2.

9. A method for monitoring the course of a viral disease in a subject infected with an influenza virus or coronavirus, said method comprising:
(a) repeatedly conducting a method as defined in any of claims 1-8 in predefined time intervals, and
(b) assigning the subject to preventive or therapeutic measures if based on the results obtained in step (a) a severe course of said viral disease is to be expected.

10. Method according to any of claims 1-9, wherein said severe course of the viral disease includes the development of the acute respiratory distress syndrome (ARDS).

11. Aromatase inhibitor for use in a method of treating or preventing the severe course of a viral disease in a subject infected with an influenza virus or coronavirus, wherein said subject has
(a) decreased testosterone levels, and/or
(b) increased estradiol levels
as compared to reference values.

12. Aromatase inhibitor for use in a method of claim 11, wherein said inhibitor is selected from the group consisting of aminoglutethimide, testolactone, anastrozole, letrozole, exemestane, vorozole, formestane, and fadrozole.

13. Aromatase inhibitor for use in a method of any of claims 11-12, wherein said severe course of the viral disease in-eludes the development of the acute respiratory distress syndrome (ARDS).

14. Testosterone or a testosterone derivative for use in a method of treating or preventing a severe course of a viral disease in a male subject infected with an influenza virus or coronavirus, wherein said male subject has (a) decreased testosterone levels compared to the normal reference levels and/or (b) increased estradiol levels compared to normal reference levels.

15. Kit for carrying out the method of any of claims 1-11, comprising:
(a) means for determining whether a subject is infected with an influenza virus or coronavirus;
(b) means for determining the concentration of testosterone and/or estradiol; and
(c) optionally, buffers and diluents.
